# EUROPEAN PATENT APPLICATION

(11) **EP 1 178 110 A1**
(43) Date of publication of application: **06.02.2002**
(21) Application number: 00116589.3
(22) Date of filing: 01.08.2000
(51) Int. Cl.: C12N 15/12, C07K 14/72, C07K 14/705, C12Q 1/68, G01N 33/68

(54) **EDG8 receptor for sphingosine 1-phosphate , its preparation and use**

(71) Applicant: Aventis Pharma Deutschland GmbH, 65929 Frankfurt am Main (DE)
(72) Inventor: Kostenis, Eva, Dr., 60594 Frankfurt Am Main (DE); Gassenhuber, Johann, 65185 Wiesbaden (DE)

(57) **Abstract**

The present invention relates to newly identified EGD8 receptors, polynucleotides encoding this receptor, polypeptides encoded by such polynucleotides, the preparation and the use of such polynucleotides and polypeptides.

## Description

The present invention relates to newly identified EGD8 receptors, polynucleotides encoding this receptor, polypeptides encoded by such polynucleotides, the preparation and the use of such polynucleotides and polypeptides.

In an effort to identify new G-protein coupled receptors of the EDG (endothelial differentiation gene)-family a novel member of the EDG-family of G-protein coupled receptors, Human EDG8, was identified. The full-length clone was isolated and studies on chromosomal mapping, tissue expression and identification as a functional cellular receptor for sphingosine 1-phosphate were performed. Taken together, the data provide compelling evidence that EDG8 is the fifth receptor for sphingosine 1-phosphate, exclusively expressed in peripheral tissues, its presence in endothelial cells being responsible for the broad tissue distribution.

The lysolipid phosphate mediators lysophosphatidic acid (LPA) and sphingosin 1-phosphate (S1P) have attracted increasing attention as modulators of a variety of important biological functions (Moolenaar et al., 1997; Morris, 1999; Lynch and Im, 1999) and their list of biological activities is continuously growing.
Among the biological responses to LPA is platelet aggregation (Jalink et al., 1994; Siess et al., 1999; Gueguen et al., 1999), smooth muscle contraction (Tokumura et al., 1980), in vivo vasoactive effects (Tokumura et al., 1995), chemotaxis (Jalink et al., 1993), expression of adhesion molecules (Lee et al., 1998b; Rizza et al., 1999), increased tight junction permeability of endothelial cells (Schulze et al., 1997), induction of stress fibers (Gohla et al., 1998) and many others (for review see Moolenaar et al., 1997). The biochemical signalling events that mediate the cellular effects of LPA include stimulation of phospholipases, mobilization of intracellular Ca²⁺, inhibition of adenylyl cyclase, activation of phosphatidylinositol 3-kinase, activation of the Ras-Raf-MAP kinase cascade and stimulation of Rho-GTPases (Moolenaar et al., 1997).

S1P, in particular, is implicated in cell proliferation, modulation of cell motility (reviewed in Hla et al., 1999) induction/suppression of apoptosis (Hisano et al., 1999; Xia et al., 1999), angiogenesis (Lee et al., 1999), tumor invasiveness (Sadahira et al., 1992), platelet activation (Gueguen et al., 1999) and neurite retraction (Postma et al., 1996). Cellular signalling by S1P involves activation of PLCβ and subsequent intracellular Ca²⁺ release (van Koppen et al., 1996; Meyer zu Heringdorf et al., 1997; Yatomi et al., 1997a; Noh et al., 1998; Ancellin and Hla, 1999), activation of MAP-kinases (Wu et al., 1995; Lee et al., 1996; An et al., 2000), activation of inward rectifying K⁺-channels (van Koppen et al., 1996; Bünemann et al., 1996) and inhibition and/or activation of adenylyl cyclase (Lee et al., 1996).
Both, LPA and S1P are recognized to signal cells through a set of G-protein coupled receptors (GPCRs) known as EDG (endothelial differentiation gene)-receptors. The EDG-family of GPCRs currently comprises seven human members (EDG1-7) that fall into two major groups depending on their preference for the activating lipid-ligand: EDG1, 3, 5 and 6 preferentially interact with S1P (Yatomi et al., 1997b; Lee et al., 1998a,b; Ancellin and Hla, 1999; Yamazaki et al., 2000; Van Brocklyn et al., 2000), EDG2, 4 and 7 preferentially interact with LPA (An et al., 1998; Im et al., 2000 ).
The assignment of specific biological functions to certain receptor subtypes is hampered by the fact that EDG receptors are expressed in an overlapping fashion (Rizza et al., 1999; Lee et al., 1999), they activate multiple and in part redundant signal transduction pathways (Lee et al., 1996; Ancellin and Hla, 1999; Kon et al., 1999; An et al., 2000), the selectivity for their activating ligands is not absolute (Lee et al., 1998b), and medicinal chemistry is only poorly developed in that specific antagonists for dissecting the pharmacology of the individual subtypes are not available yet.
An important step to shed more light on the biological role of the individual receptor subtypes would be to identify the complete set of receptors that respond to the phospholipid mediators S1P and LPA.

The present invention relates to newly identified EGD8 receptors, polynucleotides encoding this receptor, polypeptides encoded by such polynucleotides the preparation and the use of thereof.

The present invention relates to an isolated polynucleotide comprising a nucleotide sequence that has at least 90 % identity, preferably 95 % or more, most preferably 98 % identity to a nucleotide sequence encoding the polypeptide of SEQ ID NO. 2 or the corresponding fragment thereof; or a nucleotide sequence complementary to said nucleotide sequence.

Preferably, the polynucleotide is DNA or RNA. The nucleotide sequence of the polynucleotide is at least 90 % identical to that contained in SEQ ID NO. 1.; preferably 95 % or more, most preferred 98 % or more identical to SEQ ID NO. 1. In another embodiment, the polynucleotide has the nucleotide sequence SEQ ID NO. 1. In another embodiment, the polynucleotide encodes the polypeptide of SEQ ID NO. 2 or a fragment thereof. In a special embodiment, the polynucleotide is an allel of SEQ ID NO. 1. Preferably, the polynucleotide has the same essential properties and/or biological functionality as human EDG8.
One characteristic functionality is that the polynucleotid encodes for a S1P receptor; it responds to S1P and optionally also to related phospholipids like DMS 1P or LPA.

Another aspect of the invention relates to an expression system for the expression of EDG8. The EDG8 DNA or RNA molecule comprising an expression system wherein said expression system is capable of producing a polypeptide or a fragment thereof having at least 90 % identity, preferably 95 % or more, most preferred 98 % or more identity with a nucleotide sequence encoding the polypeptide of SEQ ID NO. 2 or said fragment when said expression system is present in a compatible host cell. Preferably, the expression system is a vector.
The invention relates to a host cell comprising the expression system.

In another aspect, the invention relates to a process for producing an EDG8 polypeptide or a fragment thereof wherein a host cell comprising the expression system is cultured under conditions sufficient for the production of said polypeptide or fragment thereof.
Preferably, the said polypeptide or fragment thereof is expressed at the surface of said cell.
The invention relates also to cells produced by this process.

The process preferably further includes recovering the polypeptide or fragment thereof from the culture.

In another aspect, the invention relates to a process for producing a cell which produces an EDG8 polypeptide or a fragment thereof comprising transforming or transfecting a host cell with the expression system such that the host cell, under appropriate culture conditions, produces an EDG8 polypeptide or a fragment thereof.

In particular, the invention relates to an EDG8 polypeptide or a fragment thereof comprising an amino acid sequence which has at least 90 %, preferably 95 %, most preferred 98 % or more identity to the amino acid sequence SEQ ID NO. 2 or to a part of SEQ ID NO. 2. In particular the invention relates to an EDG8 polypeptide or a fragment thereof having amino acid sequence SEQ ID NO. 2 or a part thereof. In particular, the invention relates to an polypeptide encoded by SEQ ID NO. 1 or encoded by a polynucleotide that has at least 90 %, preferably 95 %, most preferred 98 % or more identity with SEQ ID NO. 1; preferably, such polypeptid has almost the same properties as human EDG 8; e.g. the same biological functionality. One characteristic functionality of human EDG8 is that the polypeptid is a S1P receptor; it responds to S1P and optionally to related phospholipids like DMS1P or LPA.

Further, the invention relates to a process for diagnosing a disease or a susceptibility to a disease related to expression or acitivity of EDG8 polypeptide comprising:
a) determining the presence or absence of mutation in the nucleotide sequence encoding said EDG8 polypeptide in the genome of said subject; and/or
b) analyzing for the presence or amount of the EDG8 polypeptide expression in a sample derived from said subject.

In addition, the invention relates to a method for identifying compounds which bind to EDG8 polypeptide comprising:
a) contacting a cell comprising the expression system or a part of such a cell with a candidate compound; and
b) assessing the ability of said candidate compound to bind to said cells.

Preferably, the method for identifying compounds further includes determining whether the candidate compound effects a signal generated by activation of the EDG8 polypeptide at the surface of the cell, wherein a candidate compound which effects production of said signal is identified as an agonist.

In another embodiment of the invention, the method for identifying compounds further includes determining whether the candidate compound effects a signal generated by activation of the EDG8 polypeptide at the surface of the cell, wherein a candidate compound which effects production of said signal is identified as an antagonist.

The invention also relates to an agonist or antagonist identified by such methods.

In another special embodiment of the invention, the method further includes contacting said cell with a known agonist for said EDG8 polypeptide; and determining whether the signal generated by said agonist is diminished in the presence of said candidate compound, wherein a candidate compound which effects a diminution in said signal is identified as an antagonist for said EDG8 polypeptide. The known agonist is for example S1P, LPA and/or DHS1P. The invention also relates to an antagonist identified by the method.

The invention in addition, relates to a method of preparing a pharmaceutical composition comprising
a) identifying a compound which is an agonist or an antagonist of EDG8,
b) preparing the compound, and
c) optionally mixing the compound with suitable additives.
The invention also relates to a pharmaceutical compound prepared by such a process.

The invention relates to a pharmaceutical, comprising as active ingredient for example such identified compound, an EDG8 polypeptid or a polynucleotide encoding for EDG8 or a part thereof.

In particular, the invention relates to a pharmaceutical, that can be used for the prevention and/or treatment of diseases associated with EDG8/S1P signal transduction, for example diseases associated with endothelial dysfunction such as for example Atheriosclerosis, Shoke, Hypertonie, coronary syndroms, cancer, thrombolylic diseases, affected wound healing and diseases accompanied by increased cell death. In another aspect of the invention, such pharmaceutical can be used for the prevention and/or treatment of diseases associated with a dysregulation of angiogenesis, such as for example tumor growth, rheumatical arthritis and diabetic setinopathy.

The study, reported about the cloning, chromosomal mapping, tissue expression and functional identification as a receptor for S1P of a novel GPCR, EDG8, the fifth functional receptor for sphingosine 1-phosphate.

In an effort to identify new G-protein coupled receptors of the EDG-family a database search with alignments of the currently known 18 members of this receptor family was performed, comprising human EDG1-7 sequences up to the putative EDGs from Xenopus and Zebra-fish. A multiple alignment of these sequences was created by CLUSTALW and used in a PSI-BLAST search to scan translated versions of human genomic DNA sequences, which were publicly available in the different EMBL sections. For translation of DNA into protein sequences, individual protein files within two respective STOP-codon were created and all proteins shorter than 50 amino acids were ignored. As the majority of GPCRs is unspliced searching for GPCRs within genomic sequences should bring about novel receptor proteins.

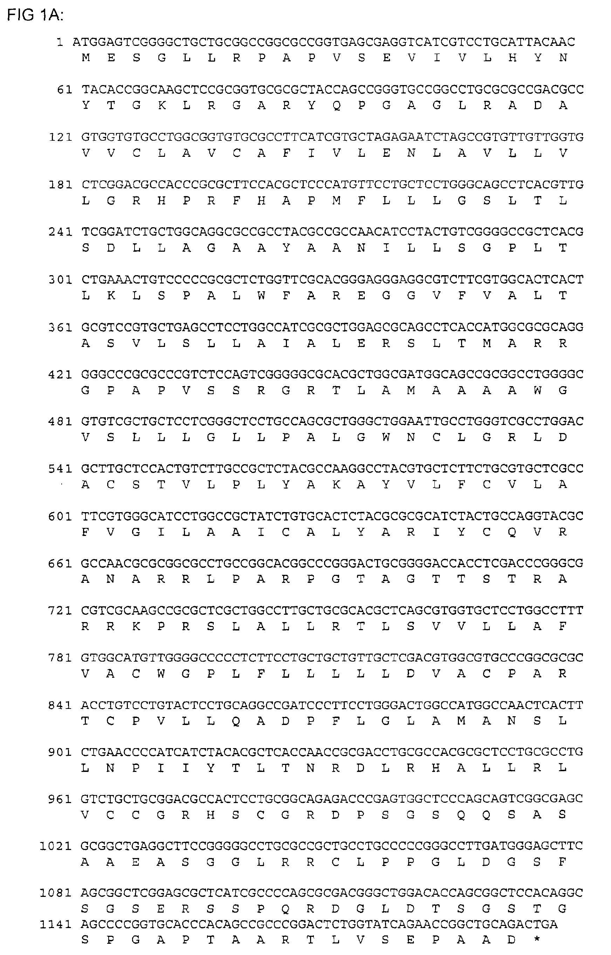

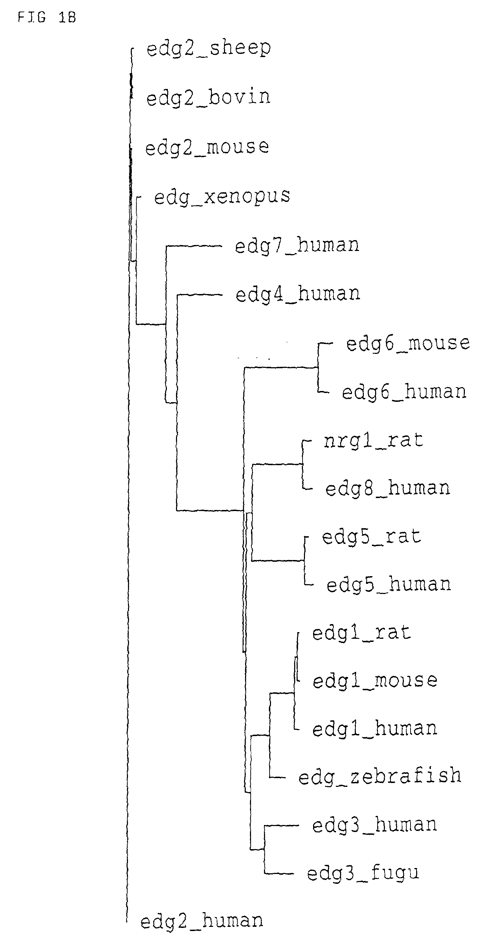

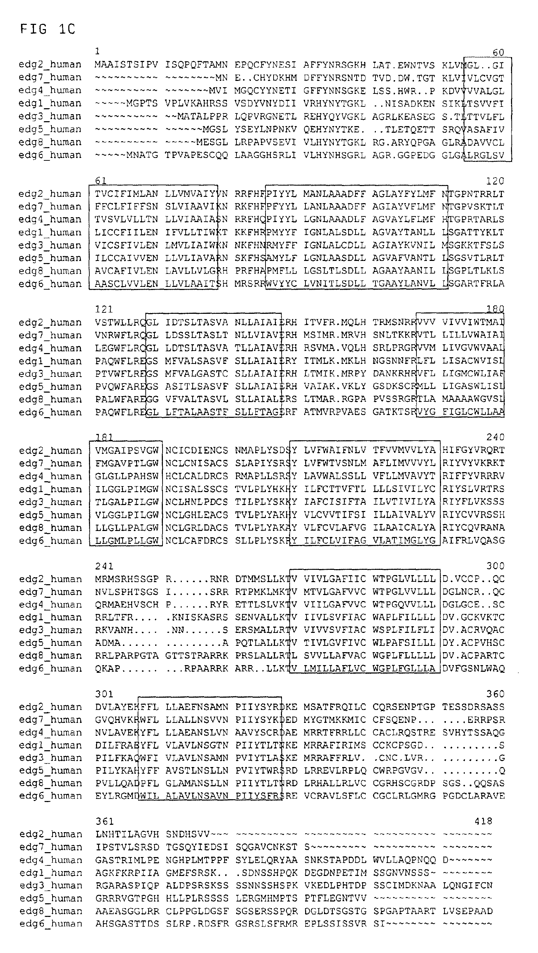

Performing a PSI-BLAST search, the various cDNAs and genomic contigs, respectively, for the human EDG1-7 receptors were identified, and an additional genomic hit, highly homologous to human EDG5 (51% homology), termed EDG8. The nucleotide and amino acid sequence of the new putative GPCR are depicted in Fig.1A. Hydropathy analysis (hydrophobicity plot not shown) suggests a seven transmembrane protein with three alternating extra- and intracellular loops, assumed to be the heptahelix structure common to GPCRs.
To shed more light on the relationships involved in the molecular evolution of the EDG-receptor family, a grow tree phylogram was constructed using the neighbor joining method (GCG software) (Fig.1B) (Comparison of amino acid sequences). According to this phylogenetic tree, the human EDG-family can be divided into two distinct groups: EDG1, 3, 5 and 6 belonging to one, EDG2, 4 and 7 belonging to the other group. These two groups are discriminated further by their preference for different lipid ligands: EDG1, 3, 5, 6 are preferentially stimulated by sphingosin 1-phosphate (S1P) (Yatomi et al., 1997b; Lee et al., 1998a,b; Ancellin and Hla, 1999; Yamazaki et al., 2000; Van Brocklyn et al., 2000), EDG2, 4 and 7 by lysophosphatidic acid (LPA) (Hecht et al., 1996; An et al., 1998; Im et al., 2000 ). The newly identified EDG8 exhibited highest similarity (86.8% aminoacid identity) to the rat nrg1-protein (Fig. 1B), a GPCR recently cloned by EST-expression profiling from a rat PC12 cell library (Glickman et al., 1999), which probably represents the rat homologue of human EDG8. In the report of Glickman, however, the authors did not address the question of the activating ligand of this receptor. The high similarity between EDG8 and the known sphingosin 1-phosphate (S1P) receptors EDG1, 3 and 5 (48-51%) (Fig. 1C) led to test the hypothesis that EDG8 may be a functional S1P-receptor.
In testing for S1P receptor activity, the EDG8 cDNA was introduced into chinese hamster ovary (CHO) cells by transient transfection. CHO cells were chosen as they exhibit minimal responses to sphingosin 1-phosphate in concentrations up to 1 µM but respond to S1P after transfection with the S1P preferring receptors EDG 1, 3 and 5 (Okamoto et al., 1998; Kon et al., 1999). To test functional receptor activity the mobilization of [Ca²⁺]ᵢ was monitored for three reasons:
1.) S1P has been reported to increase Ca²⁺ in many cell types (Ghosh et al., 1990; Zang et al., 1991; Durieux et al., 1993; Chao et al., 1994; Gosh et al., 1994; Mattie et al., 1994; Meyer zu Heringdorf et al., 1996; Okajima et al., 1996; van Koppen et al., 1996; Törnquist et al., 1997; Yatomi et al., 1997; Noh et al., 1998; An et al., 1999 )
2.) the identification of EDG1, 3, 5 and 6 as receptors for S1P has provided the molecular basis for a GPCR mediated mechanism and the receptors are known to mediate intracellular Ca²⁺-release through either PTX-sensitive Gαᵢ proteins or the PTX-insensitive Cα_{q/11} pathway (Okamoto et al., 1998; Kon et al., 1999; Gonda et al., 1999)
3.) all currently known S1P-responding EDG-receptors (except EDG6) are present in endothelial cells (A. Niedernberg et al., submitted), in which intracellular Ca²⁺ release is a major pathway in the generation of NO, an important factor in vascular biology. Thus, identification of the complete set of S1P receptors, involved in intracellular Ca²⁺ mobilization could help clarify the role of the individual subtypes in endothelial cell signalling.

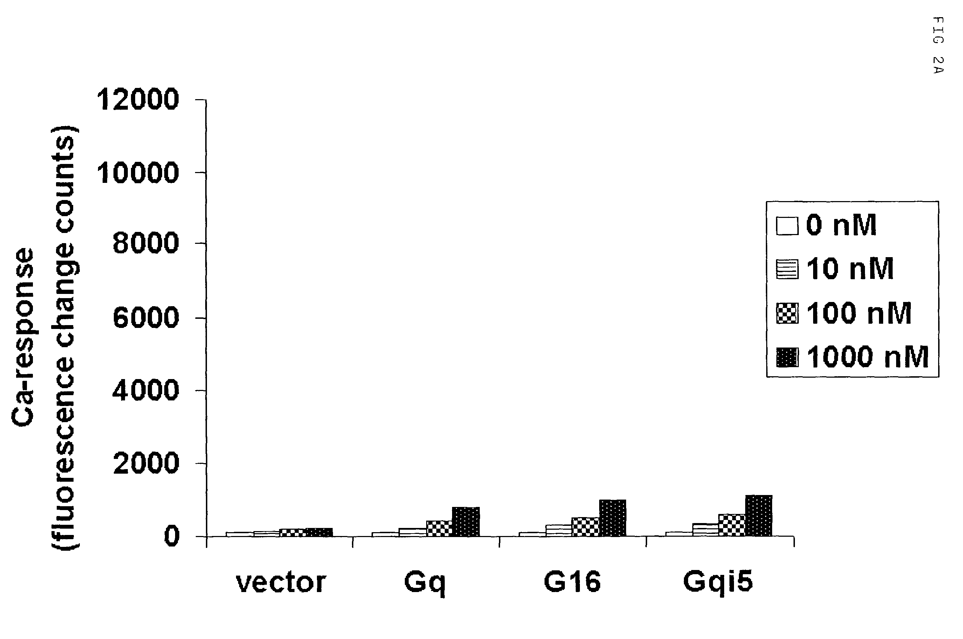

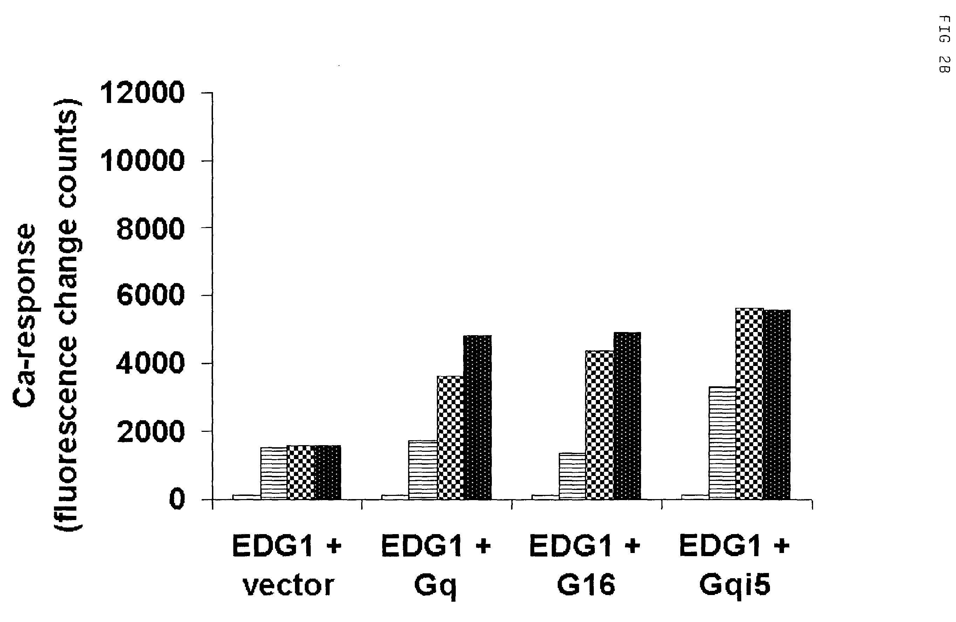

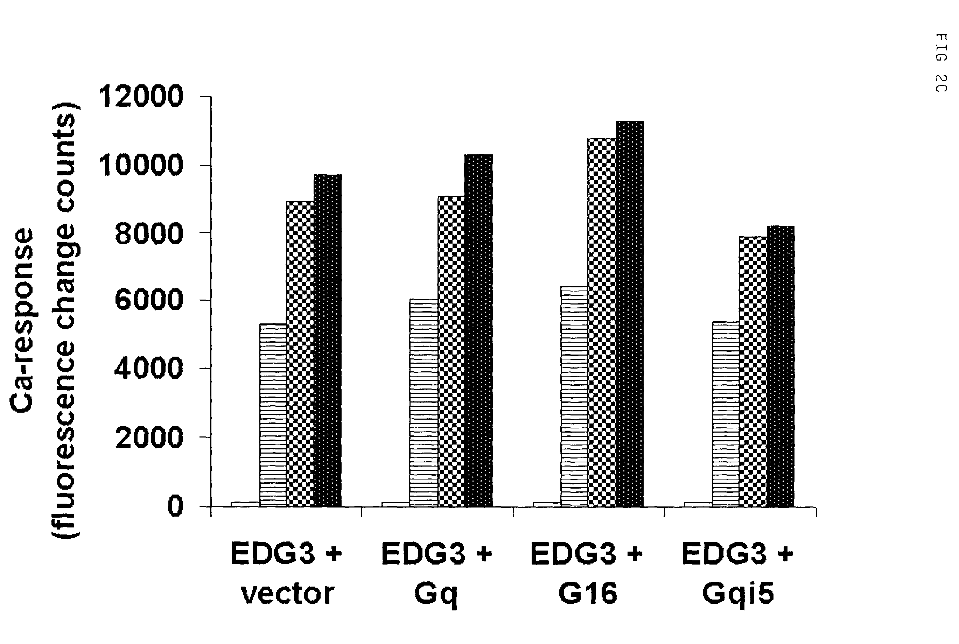

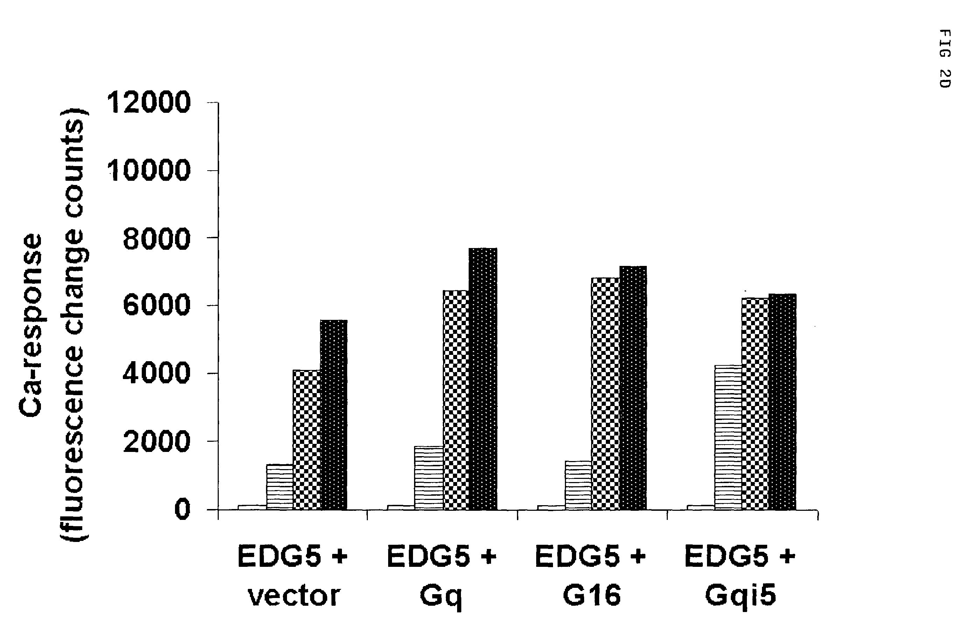

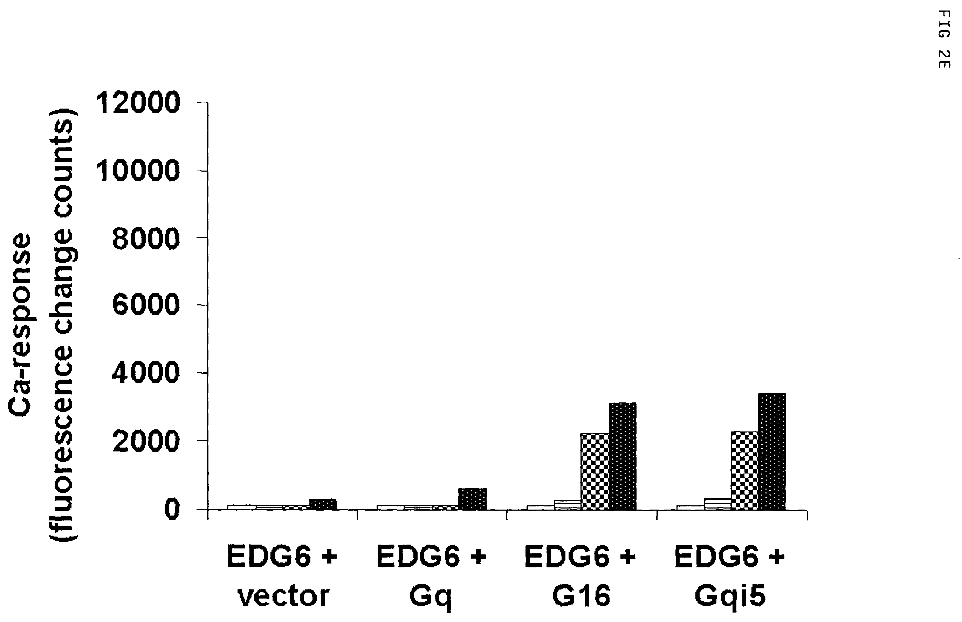

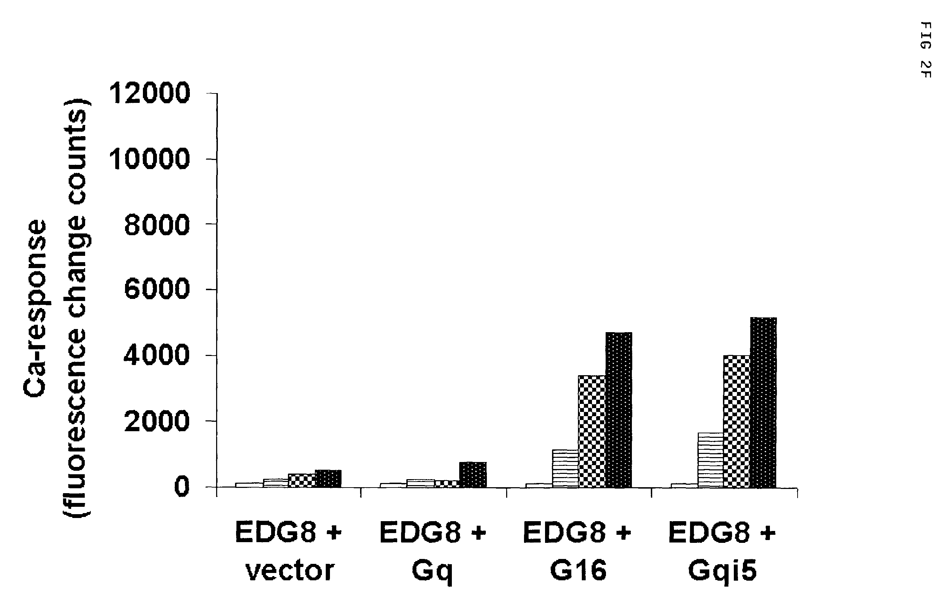

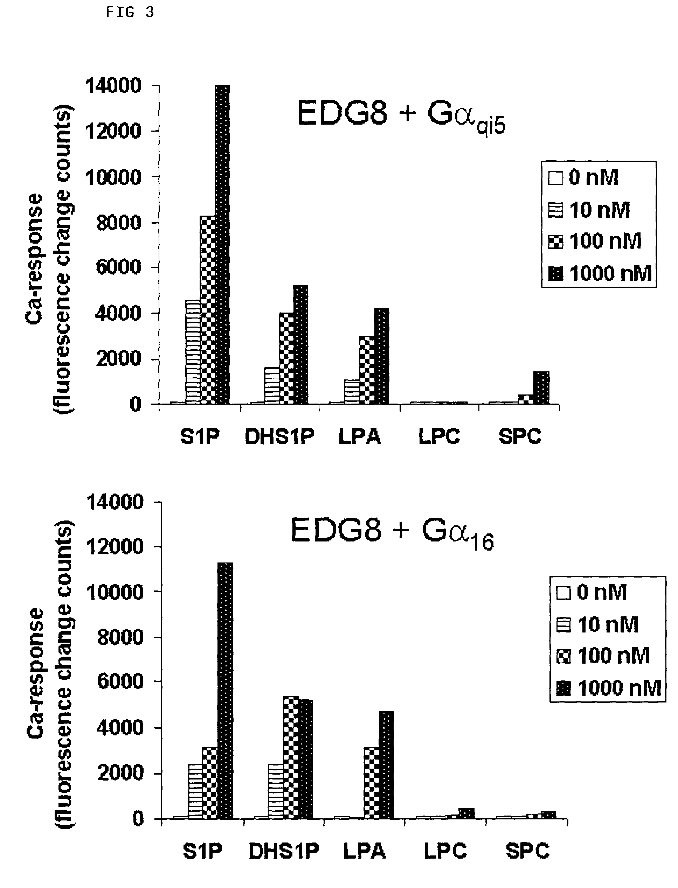

Fig.2 depicts measurement of the intracellular Ca²⁺ concentration, mediated by S1P via the putative S1P receptor EDG8. For sake of comparison, the S1P-receptors EDG1, 3, 5, and 6, which have been reported to mobilize [Ca²⁺]ᵢ, were included. [Ca²⁺]ᵢ were recorded as real time measurements using the Fluorescence plate imaging reader (FLIPR, Molecular Devices). Initially, CHO cells transfected with empty vector DNA were stimulated with different concentrations of S1P (10, 100, 1000 nM). None of the applied S1P concentrations was capable of eliciting significant rises in intracellular Ca²⁺ (Fig. 2A), suggesting that S1P receptors are not expressed in CHO cells or, if expressed, are unable to signal via the endogeneous Gα_{q} pathway. To address this issue, the G protein chimera Cα_{qi5}, which confers onto Gi coupled receptors the ability to stimulate the Gq pathway, and Cα₁₆, which links Gi- and Gs coupled receptors to PLCβ and subsequent intracellular Ca²⁺- mobilization were used. Upon stimulation with S1P, G_{qi5}- and G₁₆- transfected CHO cells did not give rise to significant increases in [Ca²⁺]ᵢ (Fig. 2A). However, transient transfection of CHO-cells with the cDNAs coding for the EDG1, 3 and 5 receptor conferred S1P-responsiveness to the cells: it was confirmed that EDG1, 3 and 5 mobilize [Ca²⁺]ᵢ in response to S1P (Fig. 2B, C, D) (Kon et al., 1999). As already known for a large number of Gq-coupled receptors, coexpression of Gα_{q} augments the EDG1 and 5-mediated Ca²⁺-response as compared with the Ca²⁺ signal induced by stimulation of endogeneous Gα_{q}. In case of EDG3, additional exogeneously added Gα_{q} did not further improve the signal intensity. These results are in agreement with the findings reported by Kon et al. (1999), who showed that the EDG3-subtype causes the most robust enhancement of intracellular Ca²⁺. In case of EDG6, Yamazaki et al. (2000) obtained an S1P-induced mobilization of [Ca²⁺]ᵢ but we failed to detect a significant Ca²⁺ increase above basal levels in the absence of any cotransfected G-protein α subunit (Fig. 2E). The reason for this discrepancy could be the cellular background (CHO cells in this study vs. K562 cells in Yamazaki et al.), as they reported a pertussis toxin (PTX)-sensitive Ca²⁺- response, indicating the involvement of Gi-type G-proteins. In this case the Ca²⁺ signal would be elicited by βγ, released from activated Gαᵢβ*γ* heterotrimers. The Gαᵢ₋ induced Ca²⁺ signals are known to be much smaller in intensity as compared with the Ca²⁺ signals induced by bona-fide Gq-linked receptors (Kostenis et al., 1997). It may be that detection of such [Ca²⁺]ᵢ concentrations is beyond the sensitivity of the FLIPR system.
EDG8 did not release [Ca²⁺]ᵢ when stimulated with S1P (10, 100, and 1000 nM) (Fig.2F), but gained the ability to mobilize Ca²⁺ upon cotransfection with Cα₁₆, a G-protein α subunit, known to couple GPCRs from different functional classes to the Gq-PLCβ pathway or Cα_{qi5}, a mutant G-protein α subunit that confers onto Gi-linked receptors the ability to stimulate Gq (Conklin et al., 1993). These results show that EDG8 is a functional receptor for S1P and that EDG8-induced Ca²⁺ responses are due to a non-Gq pathway, probably the activation of phospholipase Cβ2 by β*y* subunits of the Gi proteins. Furthermore, these results provide additional evidence that the S1P-preferring EDG-receptors couple differentially to the Gq and Gi pathways: EDG3 ist the most potent Ca²⁺-mobilizing receptor and overexpression of Gα_{q} does not further improve Ca²⁺ signalling; EDG1 and 5 induce moderate Ca²⁺- increases, that can be significantly improved by cotransfection of Gα_{q} or a chimeric Cα_{qi5} protein; EDG8-mediated Ca²⁺-responses require cotransfection of Cα_{qi5} or Gα₁₆.
To check, whether the EDG8 receptor also reacts to related lysophospholipid mediators, we examined the abilities of lysophosphatidic acid (LPA), dihydrosphingosin 1-phosphate (DHS1P), sphingosylphosphorylcholine (SPC) and lysophosphatidylcholine (LPC) to increase intracellular Ca²⁺ in CHO cells transiently transfected with the EDG8 receptor and the G-protein *α* subunits Gα₁₆ and Cα_{qi5} (Fig.3). Besides S1P, which was the most potent activator of EDG8, LPA and DHS1P evoked [Ca²⁺]ᵢ increases in concentrations of 100 and 1000 nM. SPC and LPC, respectively, failed to generate any significant response in concentrations up to 1 µM. These data show that EDG8 is a S1P preferring receptor, but also responds to related phospholipids like DHS1P or LPA, as has also been reported for EDG1, which is a high affinity receptor for S1P and a low affinity receptor for LPA (Lee et al., 1998b). Therefore, EDG8 receptor has the characteristic functionality to respond to S1P and related phospholipids like DMS 1P or LPA. The response to S1P and other related phospholipides can for example be determined as described in Example 3. Cells containing the respective Gα can be obtained as described in Example 2.

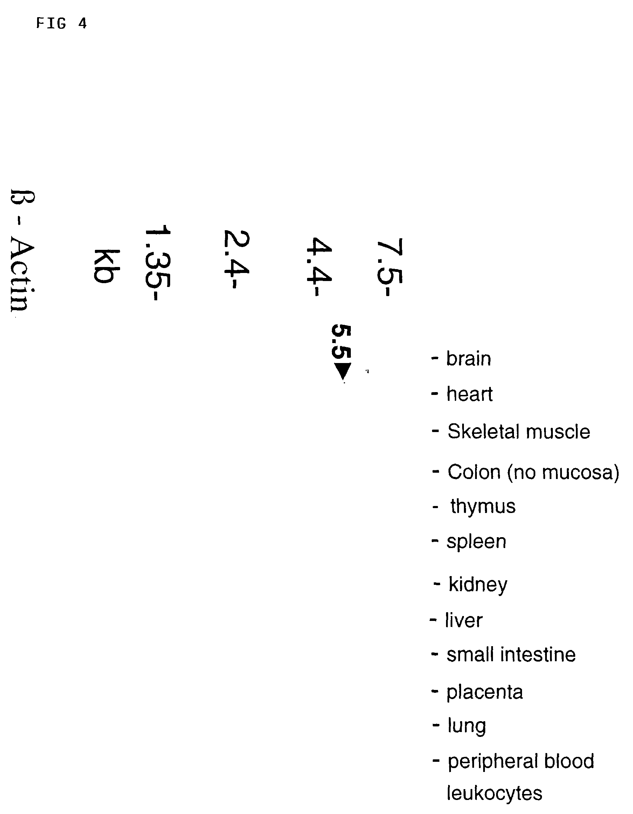

Next, the expression pattern of the EDG8 gene in human tissues was investigated by Northern blot analysis (Fig.4). Tissues positive for EDG8 RNA were skeletal muscle, heart and kidney, lower abundance of RNA was seen in liver and placenta, no signal was detected in brain, thymus, spleen, lung and peripheral blood leukocytes. In all tissues a single RNA transcript of 5.5 kb was observed after hybridization with a DIG-labelled EDG8 antisense RNA probe. EDG8 exhibits highest similarity to the rat nrg1-GPCR (Glickman et al., 1999) with an amino acid identity of 86.8% (Fig.1B) suggesting that it may be the human homolog of the rat nrg1 protein. However, the expression pattern of human EDG8 is quite different from the rat nrg1-receptor, which is found almost exclusively in brain (Glickman et al., 1999). This finding suggests that EDG8 may represent a closely related but entirely different receptor from nrg1, rather than the human homolog. Never the less, it does not rule out the possibility that EDG8 and nrg1 are homologs with entirely different, species-dependent expression patterns.

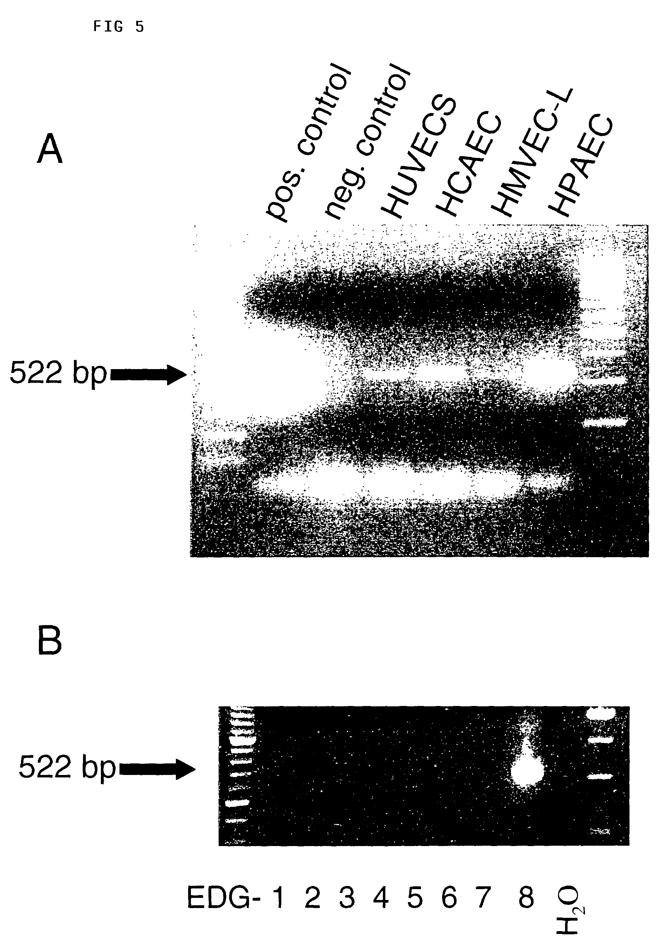

As the first member of the EDG-family of GPCRs - EDG1 - was originally cloned as an endothelial differentiation gene from phorbol-myristic-acetate-treated differentiating human endothelial cells (Hla and Maciag, 1990) and subsequently cloned from a human umbilical vein endothelial cell library exposed to fluid shear stress as an upregulated gene it is reasonable to assume that EDG receptors play an important role in the regulation of endothelial function. Therefore, the presence of EDG8 transcripts in several human endothelial cell lines was analyzed. RT-PCR analysis of human umbilival vein endothelial cells (HUVECs), human coronary artery endothelial cells (HCAECs), human microvascular endothelial cells of the lung (HMVEC-L) and human pulmonary artery endothelial cells (HPAEC) revealed EDG8 expression in all cell lines tested (Fig.5A). In Fig.5B it is shown that EDG8 specific primers indeed solely amplify EDG8 sequences and none of the related EDG1-7 sequences. These findings suggest that the presence of EDG8 in different peripheral organs may be due to its localization in endothelial cells; it does not rule out, however, that EDG8 transcripts occur in cell types other than endothelial cells.
The expression of EDG8 in addition to EDG1, 3, and 5 (Rizza et al., 1999) in HUVECS and several other endothelial cell lines is intriguing in view of all the reports regarding S1P effects on endothelial cell signalling. Hisano et al. (1999) reported that S1P protects HUVECS from apoptosis induced by withdrawal of growth factors and stimulates HUVEC DNA synthesis; the authors derived a model for cell-cell interactions between endothelial cells and platelets but the S1P-receptor responsible for HUVEC-protection of apoptosis could not be identified. Rizza et al., 1999 reported that S1P plays a role in endothelial cell leukocyte interaction in that S1P induces expression of cell adhesion molecules in human aortic endothelial cells, allowing monocytes and neutrophils to attach. These effects were blocked by pertussis toxin, suggesting the involvement of a Gi-coupled S1 P receptor. The responsible S1P-receptor subtype, however, could not be identified and the EDG8 receptor was not included at the time of this study. Expression profiling of all EDG receptors in individual cell lines and the use of EDG receptor subtype selective compounds will clearly be necessary to help determine the role of the individual S1P receptors in endothelial cell signalling mechanisms.

Finally, the mapping of EDG receptors in genomic sequences allowed to derive the chromosomal localization for four genes of this family (Tab.1). Interestingly, so far, four EDG-receptors including EDG8 are located on chromosome 19. In addition, the genomic sequence allowed the determination of the structure of the genes: the S1P-preferring receptors EDG1, 3, 5 and 8 are intronless as opposed to the LPA-preferring subtypes 2, 4 and 7, that contain an intron in the open reading frame in TMVI. These data suggest that in addition to the activating ligand and the degree of homology, the two subclasses of lysophospholipid receptors can be discriminated further by their genomic structure. The genomic structure of new potential EDG/LPA-receptor family members may also help predict the nature of the activating lipid ligand.

In conclusion, a new member of the EDG-family of G-protein coupled receptor, human EDG8, was isolated. This receptor functions as a cellular receptor for sphingosine 1-phosphate. EDG8 could exclusively be detected in peripheral tissues like skeletal muscle, heart and kidney and several human endothelial cell lines. It is conceivable that the expression in endothelial cells may account for the broad tissue distribution of this receptor. The existence of at least eight EDG-receptors for lysophospholipids suggests that receptor subtype selective agonists and antagonists will essentially be necessary for a better understanding of the biology of lysophospholipids and their respective receptors.

### Figure legends

Fig.1A: The nucleotide and deduced amino acid sequence of human EDG8. The deduced amino acid sequence is shown below the nucleotide sequence with the nucleotide positions indicated on the left.

Fig. 1B: Phylogenetic tree of the EDG-family of receptors. The phylogenetic tree depicted was derived by the neighbor joining method method performed with the GCG program.

Fig.1C: Alignment of the amino acid sequence of human EDG8 with the other EDG-family members. The amino acid sequence of EDG8 is compared with the EDG1-7 polypeptides (EDG1: accession number M 31210, EDG2: accession number U 80811, EDG3: accession number X 83864, EDG4: accession number AF 011466, EDG5: accession number AF 034780, EDG6: AJ 000479, EDG7: accession number AF 127138). The approximate boundaries of the seven putative transmembrane domains are boxed. Gaps are introduced to optimize the alignment.

Fig.2A-F: Mobilization of intracellular Ca²⁺ by S1P (10, 100 and 1000 nM) mediated by the EDG1, 3, 5, 6 and 8 receptor in CHO cells, cotransfected with empty vector DNA as a control or the indicated G-protein α subunits.
A: S1P-induced Ca²⁺-response in CHO cells transfected with vector DNA alone or the G protein α subunits Gq, G16 and Gqi5. B-F: S1P-induced Ca²⁺-response in CHO cells transfected with the indicated EDG-receptor subtypes. Agonist-mediated changes of intracellular Ca²⁺ were measured with the FLIPR using the Ca²⁺- sensitive dye FLUO4 as described in Experimental procedures. Fluorescence of transfected cells loaded with FLUO4 was recorded before and after addition of S1P, applied in the indicated concentrations. Data are expressed as means of quadruplicate determinations in a single experiment. An additional experiment gave similar results.

Fig.3: Effects of S1P, LPA and related lysophospholipid mediators on EDG8-mediated increase in intracellular Ca²⁺. CHO-cells were cotransfected with EDG8 and the G protein α subunits Gqi5 (upper panel) and G16 (lower panel) and rises in [Ca²⁺]ᵢ were recorded with the FLIPR as described in Experimental procedures. The different lipids were applied in concentrations of 10, 100 and 1000 nM, respectively. Data are means of quadruplicate determinations of a representative experiment. Two additional experiments gave similar results.

Fig.4: Northern blot analysis of EDG8 in human tissues. Poly(A)+ RNA (1µg) from various human tissues (human multiple tissue Northern blots, CLONTECH) was hybridized with probes specific to human EDG8 (upper panel) and β-actin (lower panel) on a nylon membrane. The origin of each RNA is indicated at the top, the molecular mass of standard markers in kilobases (kb) is shown on the left.

Fig.5A: Reverse transcriptase-polymerase chain reaction (RT-PCR) analysis of EDG8 in different human endothelial cell lines (HUVECS: human umbilical vein endothelial cells; HCAEC: human coronary artery endothelial cells; HMVEC-L: human microvascular endothelial cells from lung; HPAEC: human pulmonary artery endothelial cells). EDG8-specific transcripts were detected in all endothelial cell lines. Agarose gel electrophoresis of the PCR products after 35 cycles of amplification with the GC-melt kit (as described in Experimental Procedures) is shown. Amplification with EDG8-specific primers yields a 522 bp EDG8-fragment as indicated by the arrow. The EDG8 plasmid served as a template for the positive control, H₂O was used instead of plasmid DNA as a negative control.

Fig.5B: PCR analysis of EDG8 primers for specificity of amplification of EDG8 sequences. Primers, specific for the EDG8 sequence, were checked for potential amplification of the related EDG1-7 sequences, using the respective plasmids as templates. Agarose gel electrophoresis of the PCR products after 35 cycles of amplification with the GC-melt kit (as described in Experimental Procedures) is shown. The EDG8 specific 522 bp band occurred only when EDG8 was used as a template. H₂O was used instead of plasmid DNA as a negative control.

TABLE 1: Chromosomal localization, gene structure and accession number of the respective EDG genomic clones
Mapping of EDG receptors in genomic sequences allowed to derive a chromosomal assignment for EDG1, 2, 4-8. The chromosomal localization of EDG3 was obtained from Yamagutchi et al. (1996). Genomic sequences also revealed EDG1, 3, 5, 6 and 8 to be unspliced as opposed to EDG2, 4 and 7, which contain an intron in their open reading frame (ORF).

| EDG | Chromosomal localisation spliced/unspliced in ORF | | according BAC accession number: |
|---|---|---|---|
| EDG1 | 1p21.1-21.3 | unspliced | AL161741 |
| EDG2 | 9q31.1-32/ /18p11.3 | spliced | AL157881/ /AP000882 |
| EDG3 | 9q22.1-q22.2 | unspliced | |
| EDG4 | 19p12 | spliced | NT_000939 |
| EDG5 | 19 | unspliced | AC011511 |
| EDG6 | 19p13.3 | unspliced | AC011547 |
| EDG7 | 1p22.3-31.2 | spliced | AL139822 |
| EDG8 | 19 | unspliced | AC011461 |

### Examples

### Example 1: Molecular cloning of the human EDG8 receptor.

As the putative human EDG8 sequence is intronless, we cloned the receptor from human genomic DNA (CLONTECH, Palo Alto, CA, 94303-4230) via polymerase chain reaction (PCR). PCR conditions, established to amplify the EDG8 sequence were 94°C, 1 min followed by 35 cycles of 94°C, 30sec, 68C, 3 min, using GC-Melt Kit (CLONTECH, Palo Alto,CA). Primers designed to amplify the EDG8 sequence contained a HindIII site in the forward, and a EcoRI site in the reverse primer, respectively. The 1197 bp PCR product was cloned into the pCDNA3.1(+) mammalian expression vector (Invitrogen, Carlsbad, California) and sequenced in both directions.

### Example 2: Cell culture and Transfection.

CHO-K1 cells were grown in basal ISCOVE medium supplemented with 10% fetal bovine serum at 37°C in a humidified 5% CO2 incubator. For transfections, 2 x 10⁵ cells were seeded into 35-mm dishes. About 24 hr later cells were transiently transfected at 50-80% confluency with the indicated receptor and G-protein constructs (1µg of plasmid DNA each) using the Lipofectamine transfection reagent and the supplied protocol (GIBCO). 18-24 hr after transfection cells were seeded into 96well plates at a density of 50.000 cells per well and cultured for 18-24 additional hr until used in the functional FLIPR assays.
The cDNA for Gα16 was cloned from TF1 cells by RT-PCR and ligated into the pCDNA1.1 mammalian expression vector (Invitrogen). Murine wild type Gαq was cloned from cells by RT-PCR and inserted into the *Bam*Hl-*Nsi*l-sites of pCDNA1.1. To create the C-terminally modified Gα_{qi5} subunit, in which the last five aa of wt Gαq were replaced with the correspoding Gαᵢ sequence, a 175-bp *Bg*/II-*Nsi*I fragment was replaced, in a two piece ligation, with a synthetic DNA fragment, containing the desired codon changes. The correctness of all PCR-derived sequences was verified by sequencing in both directions.

### Example 3: Fluorometric Imaging Plate Reader (FLIPR) Assay.

Twenty-four hours after transfection, cells were splitted into 96-well, black-wall microplates (Corning) at a density of 50,000 cells per well. 18-24 hr later, cells were loaded with 95µl of HBSS containing 20 mM Hepes, 2.5 mM probenecid, 4 µM fluorescent calcium indicator dye Fluo4 (Molecular Probes) and 1% fetal bovine serum for 1 h(37°C, 5% CO₂). Cells were washed three times with HBSS containing 20 mM Hepes and 2.5 mM probenecid in a cell washer. After the final wash, the solution was aspirated to a residual volume of 100 µl per 96 well. Lipid ligands were dissolved in DMSO as 2 mM stock solutions (treated with ultrasound when necessary) and diluted at least 1:100 into HBSS containing 20 mM HEPES, 2.5 mM probenecid and 0.4 mg/ml fatty acid free bovine serum albumine. Lipids were aliquoted as 2X solutions into a 96 well plate prior to the assay. The fluorometric imaging plate reader (FLIPR, Molecular Devices) was programmed to transfer 100 µl from each well of the ligand microplate to each well of the cellplate and to record fluorescence during 3 min in 1 second intervals during the first minute and 3 second intervals during the last two minutes. Total fluorescence counts from the 18-s to 37-s time points are used to determine agonist activity. The instrument software normalizes the fluorescent reading to give equivalent initial readings at time zero.

### Example 4: Northern Blot analysis.

Human multiple tissue Northern blots were purchased from CLONTECH (Palo Alto, CA, 94303-4230, USA) antisense RNA probes were generated by subcloning nucleotides 279-1197 of the coding region into the Bam HI-Eco RI sites of the expression vector PSPT18 (Roche Diagnostics, Mannheim, Germany) and subsequent random priming with a DIG-RNA Labeling kit (Roche Diagnostics, Mannheim, Germany), using T7 RNA polymerase. Hybridization was carried out at 68°C for 16 h in hybridization buffer (Dig Easy Hyb Roche Diagnostics, Mannheim, Germany). Each blot was washed , blocked and detected as indicated in the standard protocol with the DIG Wash and Block Buffer set (Roche Diagnostics, Mannheim, Germany) and treated with 1 ml CSPD ready-to-use(Roche Diagnostics, Mannheim, Germany) for 15 min , 37°C and developed for 5 min on the Lumiimager (Roche). Finally, each blot was stripped (50 % formamid,5% SDS, 50 mM Tris/HCl pH 7,5 ; 80° C, 2x 1 hour) and rehybridized with a GAPDH antisense RNA probe as an internal standard.

### Example 5: RNA Extraction and RT-PCR.

RNA was prepared from different endothelial cell lines (HUVECS, HCAEC, HMVEC-L, HPAEC) using the TRIzol reagent (Hersteller, Lok.). Briefly, for each endothelial cell line, cells of a subconfluent 25 cm2 tissue culture flask were collected in 2,5ml TRIzol and total RNAs were extracted according to the supplied protocol. The purity of the RNA preparation was checked by veryfying the absence of genomic DNA. An aliquot of RNA, corresponding to ∼5µg, was used for the cDNA generation using MMLV reverse transcriptase and the RT-PCR kit from STRATAGENE. RT-PCR was carried out in a volume of 50 µl, the RT-PCR conditions were set to 65°C for 5 min, 15min at RT, 1 hour at 37°C, 5 min at 90°C, chill on ice.
The cDNA templates for the PCR reactions (35 cycles of 94°C for 30 sec, 68°C for 3 min) were the reverse transcribed products of RNAs isolated from human endothelial cell lines (HUVECS,HCAEC, HMVEC-L, HPAEC). Typically, 1-5 µl of reverse transcribed cDNAs were used as templates for the PCR reactions.

### Example 6: Sources of materials.

1-oleoyl-LPA, sphingosin 1-phosphate (S1P), dihydrosphingosin 1-phosphate (DHS1P), lysophosphatidylcholine (LPC), sphingosylphosphorylcholine (SPC) and fatty acid free BSA were from SIGMA (P.O.Box 14508, St. Louis, Missouri 63178). CHO-K1 cells were obtained from the American Type culture collection (ATCC, Manassas, Virginia), cell culture media and sera from GIBCO BRL (Gaithersburg, MD), the Ca fluorescent dye FLUO4 and pluronic acid from Molecular devices (Sunnyvale CA 94089-1136,USA) human northern blot membrane from CLONTECH (1020 East Meadow Circle, Palo Alto, California 94303-4230, USA.), commercially available cDNAs (heart, fetal heart, left atrium, left ventricle, kidney, brain, liver, lung, aorta) from Invitrogen, oligonucleotides from MWG-Biotech AG (Ebersberg, Germany), the RT-PCR kit from SIGMA, the GC-melt PCR kit from Clontech (Palo Alto, CA), the expression plasmid pcDNA3.1 for EDG8 and pCDNA1.1 for expression of G-protein α subunits from Invitrogen (Carlsbad, CA 92008), competent DH5α from GIBCO and MC 1063 from Invitrogen.

### References

An S, BleuT, Hallmark OG, and Goetzl EJ (1998) Characterization of a novel subtype of human G protein coupled receptor for lysophosphatidic acid. *J Bio. Chem* 273:7906-7910
An S, Bleu T, and Zheng Y (1999) Transduction of intracellular calcium signals through G protein-mediated activation of phospholipase C by recombinant sphingosine 1-phosphate receptors. *Mol Pharmaco.* 55:787-794
An S, Zheng Y, and Bleu T (2000) Sphingosine 1-phosphate induced cell proliferation, survival, and related signaling events mediated ba G protein coupled receptors edg3 and edg5. *J Biol Chem* 275:288-296
Ancellin N and Hla T (1999) Differential pharmacological properties and signal transduction of the sphingosine 1-phosphate receptors EDG-1, EDG-3, and EDG-5. J*J Biol Chem* 274:18997-19002
Bandoh K, Aoki J, Hosono H, Kobayashi S, Kobayashi T, Murakami-Murofushi K, Tsujimoto M, Arai H, and Inoue K (2000) *J Biol Chem* 274:27776-27785
Bünemann M, Liliom K, Brandts BK, Pott L, Tseng JL, Desiderio DM, Sun G, Miller D, and Tigyi G (1996) A novel membrane receptor with high affinity for lysosphingomyelin and sphingosine 1-phosphate in atrial myocytes. *EMBO J* 15:5527-5534
Chao CP, Laulederkind SJ, and Ballou LR (1994) Sphingosine mediated phosphatidyl metabolism and calcium mobilization. *J Biol Chem* 269:5849-5856
Durieux ME, Carlisle SJ, Salafranca MN, and Lynch KR (1993) Responses to sphingosine 1-phosphate in X. laevis oocytes: similarities with lysophosphatidic acid signalling. *Am J Physiol* 264:C1360-C1364
Glickman M, Malek RL, Kwitek-Black AE, Jacob HJ, and Lee NH (1999) Molecular cloning, tissue-specific ecpression, and chromosomal localization of a novel nerve growth factor-regulated G-protein-coupled receptor, nrg-1. *Molecular and Cellular Neuroscience* 14:141-152
Gohla A, Harhammer R, and Schultz G (1998) The G protein G13 but not G12 mediates signalling from lysophosphatidic acid receptor via epidermal growth factor to Rho..
   *J Biol Chem* 273:653-4659
Gohla A, Offermanns S, Wilkie TM, and Schultz G (1999) Differential involvement of G□12 and G□13 in receptor-mediated stress fiber formation. *J Biol Chem* 274:17901-17907
Gonda K, Okamoto H, Takuwa N, Yatomi Y, Okazaki H, Sakrai T, Kimura S, Sillard R, Harii K, and Takuwa Y (1999) The novel sphingosine 1-phosphate receptor AGR16 is coupled via pertussis toxin-sensitive and -insensitive G-proteins to multiple signalling pathways. *Biochem J* 337:67-75
Gosh TK, Bian J, and Gill DL (1994) Sphingosine 1-phosphate generated in the endoplasmic reticulum membrane activates release of stored Calcium. *J Biol Chem* 269:22628-22635
Gueguen G, Gaige B, Grevy JM, Rogalle P, Bellan J, Wilson M, Klaebe A, Pont F, Simon MF, and Chap H (1999) Structure-activity analysis of the effects of lysophosphatidic acid on platelet aggregation. *Biochemistry* 38:8440-8450
Hisano N, Yatomi Y, Satoh K, Akimoto S, Mitsumata M, Fujino MA, and Ozaki Y (1999)
   Induction and suppression of endothelial cell apoptosis by sphingolipids: a possible in vitro model for cell-cell interactions between platelets and endothelial cells.
   *Blood* 93:4293-4299
Hla T and Maciag T (1990) An abundant transcript induced in differentiating human endothelial cells encodes a polypeptide with structural similarities to G-protein-coupled receptors. *J*. *Biol. Chem.* 265: 9308-9313
Hla T, Lee M, Ancellin N, Liu CH, Thangada S, Thompson BD, and Kluk M (1999) Sphingosine-1-phosphate: extracellular mediator or intracellular second messenger? *Biochem Pharm* 58:201-207
Im DS, Heise CE, Harding MA, George SR, O'Dowd BF, Theodorescu D, and Lynch KR (2000) Molecular cloning and characterization of a lysophosphatidic acid receptor, edg7, expressed in prostate. *Mol Pharmacol* 57:753-759
Jalink K, Moolenaar WH, and van Dujin B (1993) Lysophosphatidic acid is a chemoattractant for dictyostelium discoideum amoebae. *Proc Natl Acad Sci USA* 90:1857-1861
Jalink K, Hordijk PL, and Moolenaar WH (1994) Growth factor-like effects of lysophosphatidic acid, a novel lipid mediator. *Biochim Biophys Acta* 1198:185-196
Kon J, Sato K, Watanabe T, Tomura H, Kuwabara A, Kimura T, Tamama K, Ishizuka T, Murata N, Kanda T, Kobayashi I, Ohta H, Ui M, and Okajima F (1999) Comparison of intrinsic activities of the putative sphingosine 1-phosphate receptor subtypes to regulate several signalling pathways in their cDNA-transfected chinese hamster ovary cells. *J Biol Chem* 274:23940-23947
Kostenis E, Degtyarev MY, Conklin BR, and Wess J (1997) The N-terminal extension of G□q is critical for constraining the selectivity of receptor coupling. *J Biol Chem* 272:19107-19110
Lee MJ, Evans M, and Hla T (1996) The inducible G protein-coupled receptor edg-1 signals via the G(i)/mitogen-activated protein kinase pathway. *J Biol Chem* 271:11272-11279
Lee MJ, Van Brocklyn JR, Thangada S, Liu CH, Hand AR, Menzeleev R, Spiegel S, and Hla T (1998a) Sphingosine 1-phosphate as a ligand for the G protein coupled receptor EDG-1. *Science* 279:1552-1555
Lee MJ, Thangada S, Liu CH, Thompson BD, and Hla T (1998) Lysophosphatidic acid stimulates the G-protein-coupled receptor edg-1 as a low affinity agonist. *J Biol Chem* 273:22105-22112
Lee MJ, Thangada S, Claffey KP, Ancellin N, Liu CH, Kluk M, Volpi M, Sha'afi Rl, and Hla T (1999) Vascular endothelial cell adherens junction assembly and morphogenesis induced by sphingosine 1-phosphate. *Cell* 99:301-312
Lynch K and Im DS Life on the edg. *Trends Pharmacol Sci* 20:473-475
Mattie M, Brooker G, and Spegel S (1994) Sphingosine 1-phosphate, a putative second messenger, mobilizes Calcium from internal stores via an inositoltriphosphate-independent pathway. *J Biol Chem* 269:3181-3188
Meyer zu Heringdorf D, van Koppen CJ, Windorfer B, Himmel HM, and Jakobs KH (1997) Calcium signalling by G protein coupled sphingolipid receptors in bovine aortic endothelial cells. *Naunyn-Schmiedeberg's Arch Pharmacol* 354:397-403
Moolenaar WH, Kranenburg O, Postma FR, and Zondag GCM (1997) Lysophosphatidic acid: G-protein signalling and cellular responses. *Current opinion in cell biology* 9:168-173
Morris AJ (1999) One wheel on my wagon: lysolipid phosphate signalling. *Trends Pharmacol Sci* 20:393-395
Noh SJ, Kim MJ, Shim S, and Han JK (1998) Different signalling pathway between sphingosine 1-phosphate and lysophosphatidic acid in Xenopus oocytes: Functional coupling of the sphingosine 1-phosphate receptor to PLCx-beta in Xenopus oocytes. *J Cell Physiol* 176:412-423
Okajima F, Tomura H, Sho K, Nochi H, Tamoto K, and Kondo Y (1996) Involvement of pertussis toxin-sensitive GTP-binding proteins in sphingosine 1-phosphate induced activation of phospholipase C-Ca²⁺ system in HL60 leukemia cells.
   *FEBS Lett* 379:260-264
Okamoto H, Takuwa N, Gonda K, Okazaki H, Chang K, Yatomi Y, Shigematsu H, and Takuwa Y (1998) EDG1 is a functional sphingosine 1-phosphate receptor that is linked via a Gi/o to multiple signalling pathways, including phospholipase C activation, Ca²⁺-mobilization, ras-mitogen-activated protein kinase activation, and adenylate cyclase inhibition. *J Biol Chem* 273:27104-27110
Postma R, Jalink K, Hengeveld T, and Moolenaar WH (1996) Sphingosine 1-phosphate rapidly induces Rho-dependent neurite retraction: Action through a specific cell surface receptor. *EMBO J* 15:2388-2392
Rizza C, Leitinger N, Yue J, Fischr DJ, Wang D, Shih PT, Lee H, tigyi G, and Berliner JA (1999) *Laboratory Investigation* 79:1227-1235
Sadahira Y, Ruan F, Hakomori S, and Igarashi Y (1992) Sphingosine 1-phosphate, a specific endogeneous signalling molecule controlling cell motility and tumor cell invasiveness. *Proc Natl Acad Sci USA* 89:9686-9690
Schulze C, Smales C, Rubin LL, and Staddon JM (1997) Lysophosphatidic acid increases tight junction permeability in cultured brain endothelial cells. *J Neurochem* 68:991-1000
Siess W, Zangl KJ, Essler M, Bauer M, Brandl R, CorrinthC, Bittman R, Tigyi G, and Aepfelbacher M (1999) Lysophosphatidic acid mediates the rapid activation of platelets and endothelial cells by mildly oxidized low density lipoprotein and accumulates in human atherosclerotic lesions. *Proc Natl Acad Sci USA* 96:6931-6936
Tokumura A, Fukuzawa K, Yamada S, and Tsukatani H (1980) Stimulatory effect of lysophosphatidic acids on uterine smooth muscles of non-pregnant rats. *Arch Int Pharmacodyn Ther* 245:74-83
Tokumura A,Yotsumoto T, Masuda Y, and Tanaka S (1995) Vasopressor effect of lysophosphatidic acid on spontaneously hypertensive rats and wistar kyoto rats. *Research Communications in Molecular Patology and Pharmacology* 90:96-102
Törnquist K, Saarinen P, Vainio M, and Ahlstrom M (1997) Sphingosine 1-phosphate mobilizes sequestered Calcium, activates calcium entry, and stimulates desoxyribonucleic acid synthesis in thyroid FRTL-5 cells. *Endocrinology* 138:4049-4057
Van Brocklyn JR, Graler MH, Bernhardt G, Hobson JP, Lipp M, Spiegel S (2000) Sphingosine-1-phosphate is a ligand for the G protein-coupled receptor EDG-6. *Blood* 95(8):2624-2629
Van Koppen C, Meyer zu Heringdorf D, Laser KT, Zhang C, Jakobs KH, Bunemann M, and Pott L (1996) Activation of a high affinity Gi protein-coupled plasma membrane receptor by sphingosine 1-phosphate. *J Biol Chem* 271:2082-2087
Wu J, Spiegel S, and Sturgill TW (1995) Sphingosine 1-phosphate rapidly activates the mitogen activated protein kinase pathway by a G protein-dependent mechanism. *J Biol Chem* 270:11484-11488
Xia P, Wang L, Gamble JR, and Vadas MA (1999) Activation of sphingosine kinase by tumor necrosis factor-□ inhibits apoptosis in human endothelial cells. *J Biol Chem* 274:34499-34505
Yamazaki Y, Kon J, Sato K, Tomura H, Sato M, Yoneya T, Okazaki H, Okajima F, Ohta H(2000) Edg-6 as a putative sphingosine 1-phosphate receptor coupling to Ca(2+) signaling. *Biochem Biophys Res Commun* 268(2):583-589
Yatomi Y, Yamamura S, Ruan F, and Igarashi Y (1997a) Sphingosine 1-phosphate induces platelet activation through an extracellular action and shares a platelet surface receptor with lysophosphatidic acid. *J Biol Chem* 272:5291-5297
Yatomi Y, Igarashi Y, Yang L, Hisano N,Qi R, Asazuma N, Satoh K, Ozaki Y, and Kume S (1997b) *J Biochem (Tokyo)* 12:969-973
Zhang H, Desai NN, Olivera A, Seki T, Brooker G, and Spiegel S (1991) Sphingosine 1-phosphate, a novel lipid, involved in cellular proliferation. *J Cell Biol* 114:155-167
List of non-standard abbreviations:
   S1P, sphingosine 1-phosphate; LPA, lysophosphatidic acid; dHS1P, dihydro sphingosine 1-phosphate; SPC, sphingosylphosphorylcholine; LPC, lysophosphatidylcholine; GPCR, G-protein-coupled receptor; G-protein, guanine nucleotide-binding protein; [Ca²⁺]ᵢ, intracellular Calcium concentration, RT-PCR, reverse transcription polymerase chain reaction; bp, base pair; ORF, open reading frame; EST, expressed sequence tag; FAF-BSA, fatty acid free bovine serum albumine; HUVECS. Human umbilical vein endothelial cells; HCAEC, human coronary artery endothelial cells; HMVEC-L, human microvascular endothelial cells from lung; HPAEC, human pulmonary artery endothelial cells.

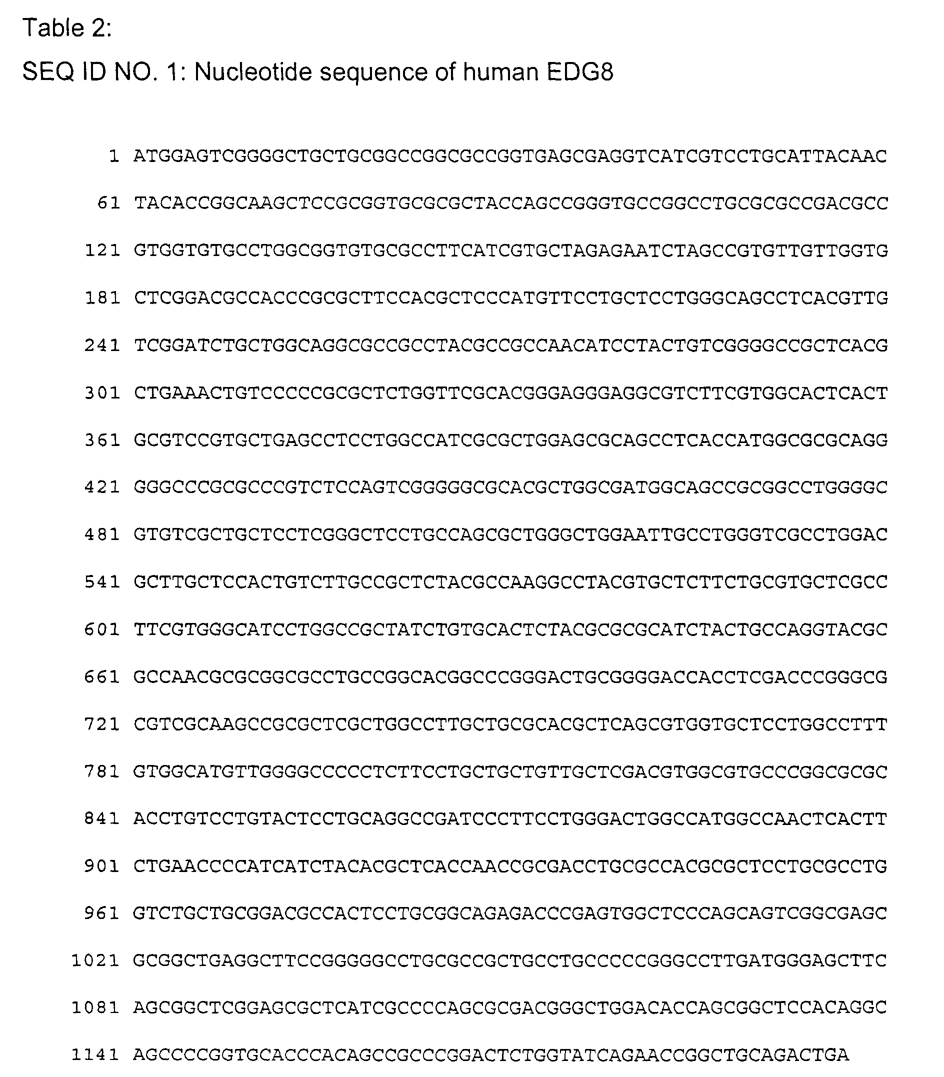

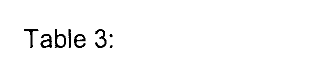

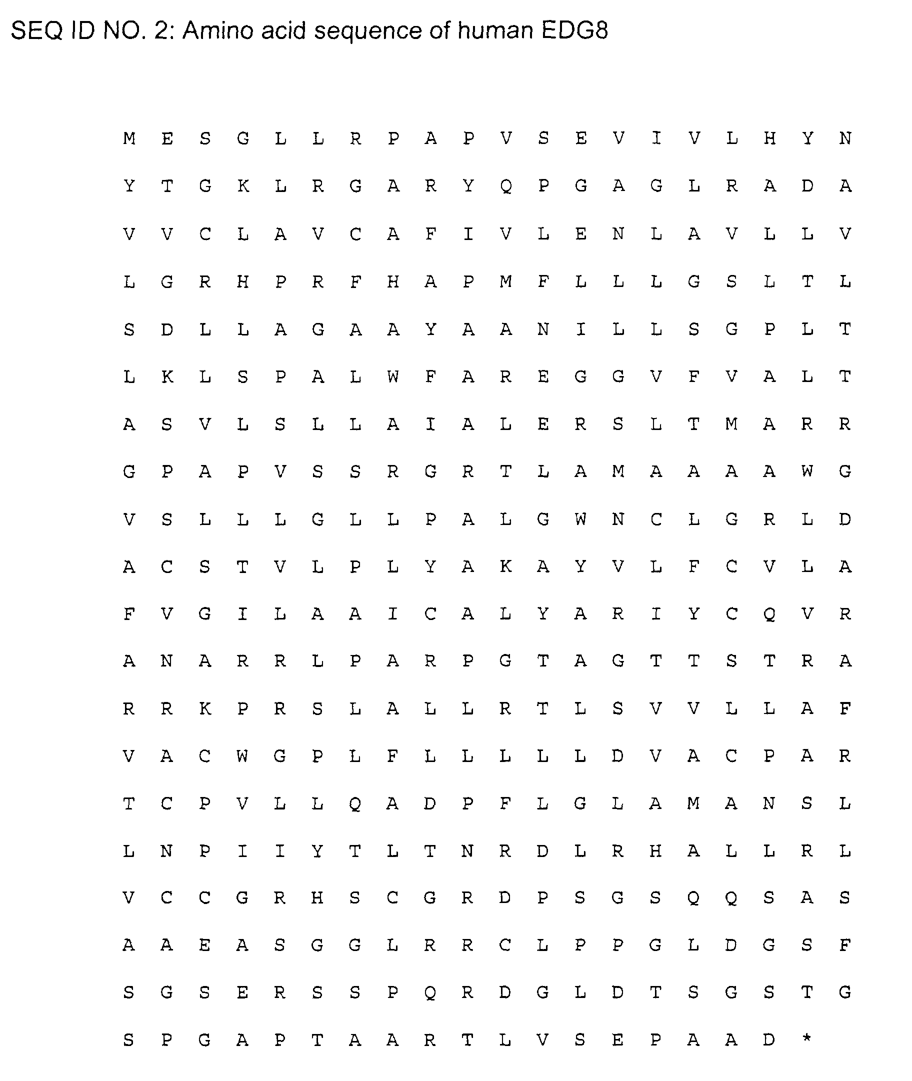

## Claims

1. An isolated polynucleotide comprising a nucleotide sequence that has at least 90 % identity to a nucleotide sequence encoding the polypeptide of SEQ ID NO. 2 or the corresponding fragment thereof; or a nucleotide sequence complementary to said nucleotide sequence.

2. The polynucleotide of claim 1 which is DNA or RNA.

3. The polynucleotide of claim 1 or 2, wherein said nucleotide sequence is at least 90 % identical to that contained in SEQ ID NO. 1.

4. The polynucleotide of claim 3 wherein said nucleotide sequence is contained in SEQ ID NO. 1.

5. The polynucleotide with sequence SEQ ID NO. 1.

6. The polynucleotide as claimed in claims 1 to 5, wherein said encoding nucleotide sequence encodes the polypeptide of SEQ ID NO. 2 or a fragment thereof.

7. The polynucleotide as claimed in claims 1 to 6 having almost the same biological functionality as EDG8.

8. EDG8 DNA or RNA molecule comprising an expression system wherein said expression system is capable of producing a polypeptide or a fragment thereof having at least 90 % identity with a nucleotide sequence encoding the polypeptide of SEQ ID NO. 2 or said fragment when said expression system is present in a compatible host cell.

9. A host cell comprising the expression system of claim 8.

10. A process for producing an EDG8 polypeptide or fragment comprising culturing a host cell as claimed in claim 9 under conditions sufficient for the production of said polypeptide or fragment.

11. The process of claim 10 wherein said polypeptide or fragment is expressed at the surface of said cell.

12. Cells produced by the process of claim 11.

13. The process of claim 10 which further includes recovering the polypeptide or fragment from the culture.

14. A process for producing a cell which produces a EDG8 polypeptide or a fragment thereof comprising transforming or transfecting a host cell with the expression system as claimed in claim 8 such that the host cell, under appropriate culture conditions, produces a EDG8 polypeptide or fragment.

15. EDG8 polypeptide or a fragment thereof comprising an amino acid sequence which is at least 90 % identical to the amino acid sequence contained in SEQ ID NO. 2.

16. Polypeptide of claim 15 which comprises the amino acid sequence of SEQ ID NO. 2, or a fragment thereof.

17. EDG8 Polypeptide or fragment prepared by the method of claim 13.

18. A process for diagnosing a disease or a susceptibility to a disease related to expression or acitivity of EDG8 polypeptide comprising:
a) determining the presence or absence of mutation in the nucleotide sequence encoding said EDG8 polypeptide in the genome of said subject; and/or
b) analyzing for the presence or amount of the EDG8 polypeptide expression in a sample derived from said subject.

19. A method for identifying compounds which bind to EDG8 polypeptide comprising:
a) tacting a cell as claimed in claim 12 or a part thereof with a candidate compound; and
b) assessing the ability of said candidate compound to bind to said cells.

20. The method as claimed in claim 19 which further includes determining whether the candidate compound effects a signal generated by activation of the EDG8 polypeptide at the surface of the cell, wherein a candidate compound which effects production of said signal is identified as an agonist.

21. The method as claimed in claim 19 which further includes determining whether the candidate compound effects a signal generated by activation of the EDG8 polypeptide at the surface of the cell, wherein a candidate compound which effects production of said signal is identified as an antagonist.

22. An agonist identified by the method of claim 20.

23. An antagonist identified by the method of claim 21.

24. The method of claim 19 which further includes contacting said cell with a known agonist for said EDG8 polypeptide; and determining whether the signal generated by said agonist is diminished in the presence of said candidate compound, wherein a candidate compound which effects a diminution in said signal is identified as an antagonist for said EDG8 polypeptide.

25. A method as claimed in claim 24, wherein the known agonist is S1P, LPA and/or DHS1P.

26. An antagonist identified by the method of claim 24 or 25.

27. Method of preparing a pharmaceutical composition comprising
a) identifying a compound which is an agonist or an antagonist of EDG8,
b) preparing the compound, and
c) optionally mixing the compound with suitable additives.

28. Pharmaceutical composition prepared by a process of claim 27.

29. Pharmaceutical composition containing an EDG8 polypeptide or a part thereof having EDG8 functionality.

30. Pharmaceutical composition containing a polynucleotide encoding for EDG8 or a part thereof encoding for a peptide with EDG8 functionality.


### Annex to the application documents - subsequently filed sequences listing

SEQUENCE LISTING

<110> Aventis Pharma Deutschland GmbH

<120> EDG8 receptor, its preparation and use

<130> AVE D-2000/A035

<140> 00116589.3
<141> 2000-08-01

<160> 2

<170> PatentIn Ver. 2.1

<210> 1
<211> 1197
<212> DNA
<213> Homo sapiens

<400> 1
atggagtcgg ggctgctgcg gccggcgccg gtgagcgagg tcatcgtcct gcattacaac 60
tacaccggca agctccgcgg tgcgcgctac cagccgggtg ccggcctgcg cgccgacgcc 120
gtggtgtgcc tggcggtgtg cgccttcatc gtgctagaga atctagccgt gttgttggtg 180
ctcggacgcc acccgcgctt ccacgctccc atgttcctgc tcctgggcag cctcacgttg 240
tcggatctgc tggcaggcgc cgcctacgcc gccaacatcc tactgtcggg gccgctcacg 300
ctgaaactgt cccccgcgct ctggttcgca cgggagggag gcgtcttcgt ggcactcact 360
gcgtccgtgc tgagcctcct ggccatcgcg ctggagcgca gcctcaccat ggcgcgcagg 420
gggcccgcgc ccgtctccag tcgggggcgc acgctggcga tggcagccgc ggcctggggc 480
gtgtcgctgc tcctcgggct cctgccagcg ctgggctgga attgcctggg tcgcctggac 540
gcttgctcca ctgtcttgcc gctctacgcc aaggcctacg tgctcttctg cgtgctcgcc 600
ttcgtgggca tcctggccgc tatctgtgca ctctacgcgc gcatctactg ccaggtacgc 660
gccaacgcgc ggcgcctgcc ggcacggccc gggactgcgg ggaccacctc gacccgggcg 720
cgtcgcaagc cgcgctcgct ggccttgctg cgcacgctca gcgtggtgct cctggccttt 780
gtggcatgtt ggggccccct cttcctgctg ctgttgctcg acgtggcgtg cccggcgcgc 840
acctgtcctg tactcctgca ggccgatccc ttcctgggac tggccatggc caactcactt 900
ctgaacccca tcatctacac gctcaccaac cgcgacctgc gccacgcgct cctgcgcctg 960
gtctgctgcg gacgccactc ctgcggcaga gacccgagtg gctcccagca gtcggcgagc 1020
gcggctgagg cttccggggg cctgcgccgc tgcctgcccc cgggccttga tgggagcttc 1080
agcggctcgg agcgctcatc gccccagcgc gacgggctgg acaccagcgg ctccacaggc 1140
agccccggtg cacccacagc cgcccggact ctggtatcag aaccggctgc agactga 1197

<210> 2
<211> 398
<212> PRT
<213> Homo sapiens

<400> 2
Met Glu Ser Gly Leu Leu Arg Pro Ala Pro Val Ser Glu Val Ile Val
1               5                   10                  15

Leu His Tyr Asn Tyr Thr Gly Lys Leu Arg Gly Ala Arg Tyr Gln Pro
            20                  25                  30

Gly Ala Gly Leu Arg Ala Asp Ala Val Val Cys Leu Ala Val Cys Ala
        35                  40                  45

Phe Ile Val Leu Glu Asn Leu Ala Val Leu Leu Val Leu Gly Arg His
    50                  55                  60

Pro Arg Phe His Ala Pro Met Phe Leu Leu Leu Gly Ser Leu Thr Leu
65                  70                  75                  80

Ser Asp Leu Leu Ala Gly Ala Ala Tyr Ala Ala Asn Ile Leu Leu Ser
                85                  90                  95

Gly Pro Leu Thr Leu Lys Leu Ser Pro Ala Leu Trp Phe Ala Arg Glu
            100                 105                 110

Gly Gly Val Phe Val Ala Leu Thr Ala Ser Val Leu Ser Leu Leu Ala
        115                 120                 125

Ile Ala Leu Glu Arg Ser Leu Thr Met Ala Arg Arg Gly Pro Ala Pro
    130                 135                 140

Val Ser Ser Arg Gly Arg Thr Leu Ala Met Ala Ala Ala Ala Trp Gly
145                 150                 155                 160

Val Ser Leu Leu Leu Gly Leu Leu Pro Ala Leu Gly Trp Asn Cys Leu
                165                 170                 175

Gly Arg Leu Asp Ala Cys Ser Thr Val Leu Pro Leu Tyr Ala Lys Ala
            180                 185                 190

Tyr Val Leu Phe Cys Val Leu Ala Phe Val Gly Ile Leu Ala Ala Ile
        195                 200                 205

Cys Ala Leu Tyr Ala Arg Ile Tyr Cys Gln Val Arg Ala Asn Ala Arg
    210                 215                 220

Arg Leu Pro Ala Arg Pro Gly Thr Ala Gly Thr Thr Ser Thr Arg Ala
225                 230                 235                 240

Arg Arg Lys Pro Arg Ser Leu Ala Leu Leu Arg Thr Leu Ser Val Val
                245                 250                 255

Leu Leu Ala Phe Val Ala Cys Trp Gly Pro Leu Phe Leu Leu Leu Leu
            260                 265                 270

Leu Asp Val Ala Cys Pro Ala Arg Thr Cys Pro Val Leu Leu Gln Ala
        275                 280                 285

Asp Pro Phe Leu Gly Leu Ala Met Ala Asn Ser Leu Leu Asn Pro Ile
    290                 295                 300

Ile Tyr Thr Leu Thr Asn Arg Asp Leu Arg His Ala Leu Leu Arg Leu
305                 310                 315                 320

Val Cys Cys Gly Arg His Ser Cys Gly Arg Asp Pro Ser Gly Ser Gln
                325                 330                 335

Gln Ser Ala Ser Ala Ala Glu Ala Ser Gly Gly Leu Arg Arg Cys Leu
            340                 345                 350

Pro Pro Gly Leu Asp Gly Ser Phe Ser Gly Ser Glu Arg Ser Ser Pro
        355                 360                 365

Gln Arg Asp Gly Leu Asp Thr Ser Gly Ser Thr Gly Ser Pro Gly Ala
    370                 375                 380

Pro Thr Ala Ala Arg Thr Leu Val Ser Glu Pro Ala Ala Asp
385                 390                 395